# EUROPEAN PATENT SPECIFICATION

(11) **EP 0 594 575 B1**
(45) Date of publication and mention of the grant of the patent: **03.02.1999**
(21) Application number: 91900397.0
(22) Date of filing: 22.11.1990
(51) Int. Cl.: A01N 25/04, A01N 37/44, C08B 11/02, A23L 3/34, A61K 7/40

(54) **MICROBICIDAL COMPOSITION CONTAINING A CELLULOSE ETHER**
MIKROBIZIDE ZUSAMMENSTELLUNG DIE EINEN CELLULOSEETHER ENTHÄLT
COMPOSITION MICROBICIDE CONTENANT UN ETHER DE CELLULOSE

(30) Priority: 22.11.1989 SE 8903920
(43) Date of publication of application: 04.05.1994
(73) Proprietor: BEROL NOBEL AB, 444 85 Stenungsund (SE)
(72) Inventor: ANDERSSON, Lars, S-444 55 Stenungsund (SE); VON CORSWANT, Christian, S-431 69 Mölndal (SE)
(74) Representative: Andersson, Rolf
(86) International application number: SE9000762
(87) International publication number: WO9107088

(56) References cited:
- WO-A-89/11503
- DE-A- 2 635 403
- DE-A- 2 929 002
- US-A- 4 790 978

## Description

The present invention relates to a microbicidal composition which contains a nonionic water-soluble cellulose ether, preferably an ethyl hydroxyethyl cellulose. In a water-base medium with pH below 6, the microbicidal composition has a prolonged microbicidal effect and an improved viscosity at elevated temperature, whereas the microbicidal effect and the improved viscosity are lost when pH is raised.

US Patent Specification 4,790,978 discloses that long-chain alkyl esters of the general formula 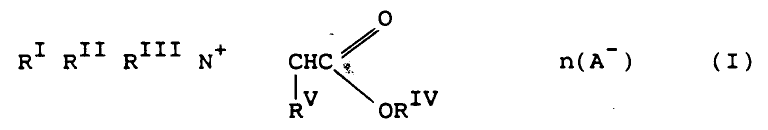 wherein R^{I}, R^{II} and R^{III} are hydrogen or lower alkyl groups, R^{IV} is a long-chain alkyl group with 10-18 carbon atoms, R^{V} is hydrogen or a group of formula

R^{VI} N⁺ H₃

wherein R^{VI} is an alkylene group with 3-4 carbon atoms, A is a monovalent counterion, and n is the number of cationic groups in the ester compound, have, in water-base systems with pH 6-8.5, a bactericidal effect which, owing to the hydrolysis of the ester bond, is temporary. The hydrolysis of the long-chain alkyl ester minimises undesirable biological effects, such as skin irritation and sensitisation. These effects have, inter alia, restricted the use of commercial quaternary ammonium compounds as disinfectants. Later, it was also found that the long-chain alkyl esters have an excellent microbicidal effect on a number of viruses, e.g. such viruses as cause venereal diseases.

When using microbicides, it is, in many cases, desirable to have available formulations in the form of high-viscous aqueous solutions or gels. It is therefore usual to add viscosity-controlling or gel-forming agents in the form of water-soluble polymers.

There are several types of gel-forming water-soluble polymers. For most of these, gelling occurs when the temperature is reduced. In aqueous solution, some polymers, such as nonionic cellulose ethers, have a so-called cloud point, which means that the polymer is precipitated when the temperature is elevated beyond a critical value. Normally, the viscosity of nonionic cellulose ethers is reduced when the temperature is raised above room temperature, and this reduction continues until the cloud point is reached. At this temperature, the solution starts to become turbid and, if the polymer content is sufficiently high, an increase in viscosity can be observed. This process is reversible.

PCT/SE89/00266 discloses that the decrease in viscosity following upon an increase in the temperature of an aqueous solution of a nonionic cellulose ether can be reduced considerably, and in many cases even turned into a considerable increase in viscosity, by using a system which has a water content of at least 85% and, in its liquid phase, contains as viscosity-controlling agents,
a) a nonionic cellulose ether in such an amount that a water solution thereof has a viscosity of 10-10,000 cP, preferably 30-5,000 cP, as measured according to Brookfield LV, 12 rpm at 20°C, and
b) a water-soluble, ionic and surface-active compound in an amount of 1-30 mmol, preferably 2-20 mmol, per litre of water.

The resulting system has a surprisingly advantageous viscosity development at elevated temperature. By adapting the cloud point of the cellulose ether and the amount of ionic surface-active compound, also gels and gel-like structures can be obtained.

One object of the present invention is to provide, for the long-chain alkyl ester compounds of formula I, a microbicidal composition containing a polymer and presenting a lower decrease in viscosity at elevated temperature than does the polymer itself and, in many cases, even presenting a maintained or increased viscosity, before any gel or gel-like structures form.

Another object of the invention is that the microbicidal composition should be stable and have a high viscosity during the entire treatment. Also, the microbicidal effect of the formulation should be high.

A further object of the invention is that the composition should be readily removable once the microbicidal treatment has been carried out. Thus, the effect of this treatment is easier to control.

Yet another object of the invention is that the removed composition should, after a short period of time, lose its microbicidal effect and only contain nontoxic ingredients.

Compositions possessing these qualities are much-needed in several areas, e.g. the food industry and the biotechnics, for disinfecting production equipment, or in therapeutical and cosmetic treatments where it may be desirable to achieve an increase in viscosity and, optionally, an ensuing gel formation upon heating to the temperature of the production equipment or to the body temperature.

Unexpectedly, it has been found that the above requirements are met by a water-base microbicidal composition containing
a) 1-30 mmol, preferably 2-20 mmol per litre of water, of a long-chain alkyl ester of the general formula I, in
b) an aqueous solution of a nonionic cellulose ether which has a viscosity of 10-10,000 cP, preferably 30-5,000 cP, as measured according to Brookfield LV, 12 rpm at 20°C, the pH of the composition being below 6, preferably 3.0-5.5.

The composition according to the invention has an advantageous viscosity development at elevated temperature, and its microbicidal effect is prolonged and remains high. When the microbicidal treatment is performed, it may be interrupted, e.g. by diluting the composition, which after the increase in temperature is high-viscous, with cold water, and/or by supplying a basic agent, such as an aqueous alkaline buffer solution. When the pH of the formulation is 6 or higher, preferably above 7, the high viscosity of the composition decreases rapidly, whereupon the composition can be easily removed. Surprisingly enough, the decrease in viscosity has been found to be much more rapid than could have been expected in view of the hydrolysis of the long-chain alkyl ester in aqueous solution. This quality is of great value since it not only makes it possible to carefully control the duration of the treatment, but also facilitates the removal of the composition. If the composition is applied to a surface emitting hydroxide ions or taking up protons, its pH will rise gradually. In such cases, it is not necessary to take any special measures for increasing the pH, but the duration of the treatment can be adjusted by the choice of a suitable pH for the composition applied.

Preferably, the alkyl groups R^{I}, R^{II} and R^{III} of the alkyl ester have 1-4 carbon atoms and are, most preferably, methyl groups. The group R^{IV} is preferably a straight-chain alkyl group. Usually, the counterion A is a halide ion, such as chloride or HSO₄⁻. Preferred embodiments of the alkyl esters include esters of the general formulae 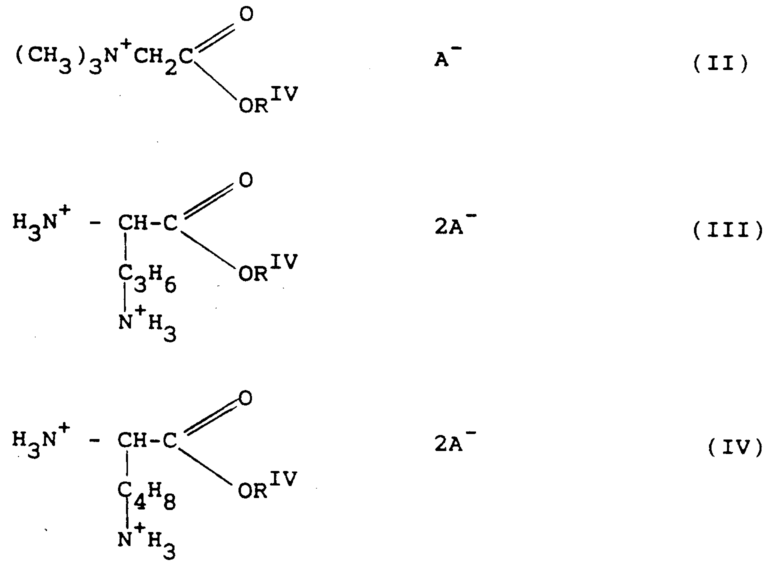 wherein R^{IV} and A are the same as above. Suitably, the alkyl ester is added in such an amount that, when gradually added to an aqueous solution containing a desired content of the cellulose ether at issue, it will increase the viscosity by at least 25%.

The cellulose ethers should be water-soluble at the temperature at which the aqueous solution is prepared. Suitably, their degree of polymerisation is such that a 1% water solution thereof has a viscosity of 10-10,000 cP, preferably 30-5,000 cP, as measured according to Brookfield LV, 12 rpm at 20°C. The cellulose ethers may comprise hydrophobic hydrocarbon groups, such as methyl, ethyl, propyl, butyl, benzyl and higher hydrocarbon groups with 8-24 carbon atoms; or polar hydroxyl groups, such as hydroxyethyl, hydroxypropyl and hydroxybutyl; or mixtures of hydrocarbon groups and polar groups. Suitably, the cloud point of the cellulose ethers is below 70° but above room temperature and is adapted to suit the field of application of the composition. Preferably, a cellulose ether is chosen whose cloud point is -5°C - +5°C of the temperature at which the composition is used. Suitable cellulose ethers include methyl cellulose, ethyl cellulose, hydroxyethyl cellulose, hydroxypropyl cellulose, methyl hydroxyethyl cellulose, ethyl hydroxypropyl cellulose, ethyl hydroxyethyl cellulose, and benzyl ethyl hydroxyethyl cellulose. Alkyl hydroxyalkyl cellulose and especially ethyl hydroxyethyl cellulose are the preferred cellulose ethers.

For topical use on human beings, compositions containing ethyl hydroxyethyl celluloses with cloud points of 30-35°C, especially 32-35°C, and viscosities of 30-400 cP in a 1% water solution, as measured according to Brookfield LV, 12 rpm at 20°C have shown excellent properties. They are easily formulated at room temperature or temperatures slightly above room temperature. After applying and distributing such a composition on the skin, a gelling effect will rapidly take place, preventing undesired running and leveling. These ethyl hydroxyethyl celluloses normally have a DS_{ethyl} of 1.2-2.5 and an MS_{hydroxyethyl} of 0.5-1.5.

The invention also relates to the preferred ethyl hydroxyethyl cellulose ethers having a cloud point of 30-35°C, especially 32-35°C. These cellulose ethers normally have a DS_{ethyl} of 1.2-2.5 and an MS_{hydroxyethyl} of 0.5-1.5, but they may also contain minor amounts of other substituents, such as methyl and hydroxypropyl. The viscosity of the ethers is preferably from 30 to 400 cP in a 1% water solution, as measured according to Brookfield LV, 12 rpm at 20°C.

The ethyl hydroxyethyl cellulose ethers may be prepared by conventional methods, for example by first mercerising cotton linters or dissolving pulp with an aqueous alkaline solution. After dewatering, if so desired, the activated cellulose is reacted with ethylene oxide at a temperature of 60-90°C and with an ethyl halide at a temperature of 95-120°C in the presence of an organic reaction medium. If desired, the mercerisation, ethoxylation, and/or alkylation may be repeated in one or more sequences. Finally, the ethyl hydroxyethyl cellulose obtained is neutralised and/or washed.

Generally, the composition according to the invention is used at a temperature exceeding the temperature at which it was prepared. In addition to pH regulators, the formulation may contain purifying agents, enzymes, bleaching agents, therapeutical and cosmetic agents, colouring agents, and salts. A high salt content should however be avoided unless a dehydrating effect is desired.

The invention will now be further illustrated by means of the following Examples.

### Example 1

Aqueous compositions were formulated from ethyl hydroxyethyl cellulose and a long-chain alkyl ester of formula

(CH₃)₃ N⁺ CH₂ COO C₁₄ H₂₉ Cl⁻

The cellulose ether had MS_{hydroxyethyl} = 1.0; DS_{ethyl} = 1.7; a cloud point of about 35°C; and a viscosity of 55 cP in a 1% water solution, as measured according to Brookfield LV, 12 rpm at 20°C.

The alkyl ester and cellulose ether contents are apparent from the Table below. The pH of the formulations was adjusted by means of HCl to 4. Then, the viscosity of the formulations was measured as a function of the temperature. The measurements were carried out by means of a Bohlin Rheometer at a shear rate of 0.2326 s⁻¹. The heating rate was 2°C per minute. The results are shown below.

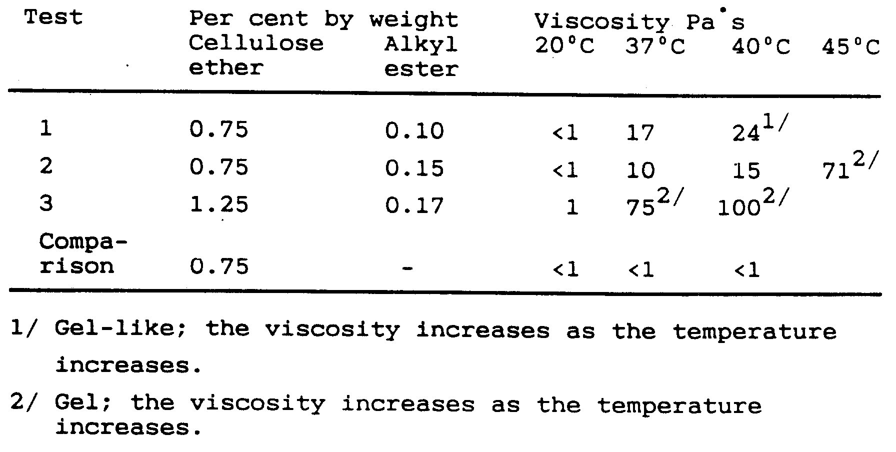

As is apparent from the results above, the viscosity of the formulation increases as the temperature increases until a gel-like or gel structure is obtained.

### Example 2

Like in Example 1, the viscosity was measured as a function of the temperature of an aqueous composition containing 1.25% by weight of the cellulose ether in Example 1 and 0.17% by weight of the alkyl ester in Example 1. pH was adjusted by means of HCl and NaOH, and the measurements began 5 minutes after the composition had been prepared. The value at 35°C was measured 10 minutes after this temperature had been reached. The following results were obtained.

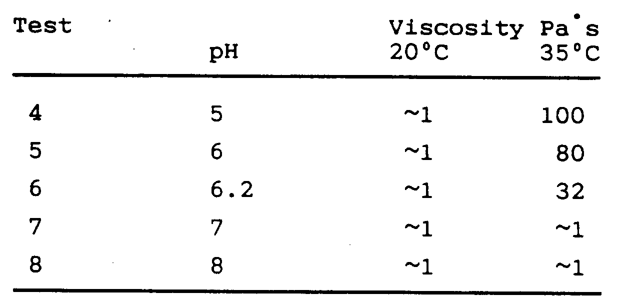

As is apparent from these results, the viscosity decreases when pH is 6 or above. This decrease is more rapid than the hydrolysis of the alkyl ester taking place in an aqueous solution at the pH at issue.

### Example 3

To 1 litre of water were added 7.5 g of the cellulose ether in Example 1 and 1.5 g of the ester compound in Example 1. To 0.4 ml of the resulting formulation was added 15 µl of a solution containing Salmonella typhimurium, whereupon the bacterial activity was measured after varying contact times. The following results were obtained.

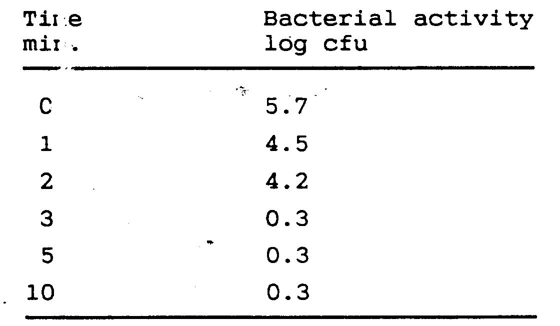

As is apparent from these results, the bactericidal effect of the composition is excellent.

### Example 4

An aqueous composition was formulated from the cellulose ether in Example 1 and a long-chain alkyl ester of formula

H₃N⁺CH(C₄H₈N⁺H₃)CO₂C₁₆H₃₃ 2Cl⁻

The alkyl ester content was 0.3% by weight, and the cellulose ether content was 0.75% by weight. The pH of the formulation was about 3.3. The viscosity of the formulation was measured at 20°C and at 35°C and was found to be <1 and 4 Pa·s, respectively. The measurements and the heating were carried out as in Example 1.

### Example 5

Aqueous compositions were formulated from ethyl hydroxyethyl cellulose and a long-chain alkyl ester, as disclosed in Example 1.

The cellulose ether had an MS_{hydroxyethyl} = 1.0, a DS_{ethyl} = 1.8, a cloud point of about 33°C and a viscosity of 67 cP in a 1% water solution, as measured according to Brookfield LV, 12 rpm at 20°C.

The alkyl ester and cellulose ether contents are apparent from the Table below. The pH of the formulations was adjusted by means of HCl to 4. Then, the viscosity of the formulations was measured as a function of the temperature. The measurements were carried out by means of a Bohlin Rheometer at a shear rate of 0.2326 s⁻¹. The heating rate was 2°C per minute. The results are shown below.

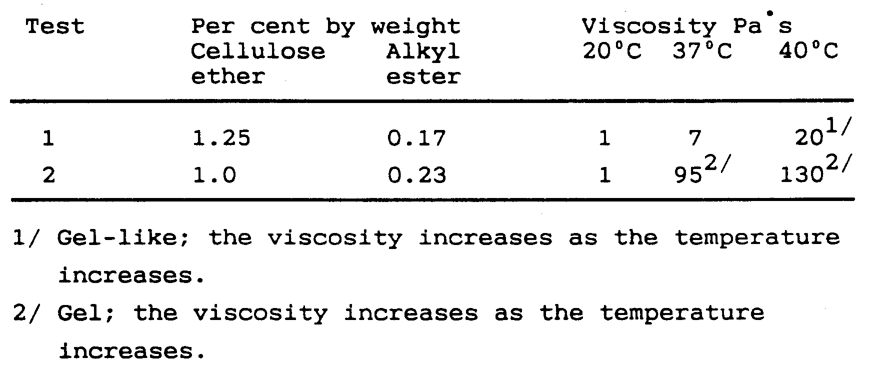

As is apparent from the results above, the viscosity of the formulation increases as the temperature increases until a gel-like or gel structure is obtained.

## Claims

1. An aqueous microbicidal composition having a microbicidal effect combined with improved viscosity, **characterised** in that it contains
a) 1-30 mmol per litre of water of a long-chain alkyl ester of the general formula 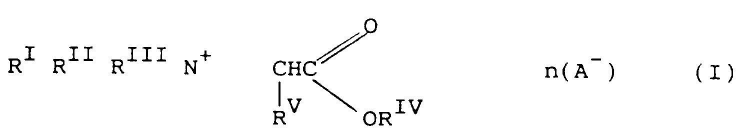 wherein R^{I}, R^{II} and R^{III} are hydrogen or lower alkyl groups, R^{IV} is a long-chain alkyl group with 10-18 carbon atoms, R^{V} is hydrogen or a group of formula
R^{VI} N⁺ H₃
wherein R^{VI} is an alkylene group having 3-4 carbon atoms, A is a monovalent counter-ion, and n is the number of cationic groups in the ester compound, in
b) an aqueous solution of nonionic water-soluble cellulose ether which has a viscosity of 10-10,000 cP as measured according to Brookfield LV, 12 rpm at 20°C, and that the composition has a pH below 6.

2. Composition as claimed in claim 1, **characterised** in that a 1% water solution of the cellulose ether has a viscosity of 10-10,000 cP.

3. Composition as claimed in any one of claims 1 and 2, **characterised** in that the cellulose ether has a cloud point below 70°C.

4. Composition as claimed in any one of claims 1-3, **characterised** in that the cellulose ether is an alkyl hydroxyalkyl cellulose.

5. Composition as claimed in claim 4, **characterised** in that the cellulose ether is an ethyl hydroxyethyl cellulose having a cloud point of 30-35°C.

6. Composition as claimed in claim 5, **characterised** in that the cellulose ether has a DS_{ethyl} of 1.2-2.5 and an MS_{hydroxyethyl} of 0.5-1.5.

7. Composition as claimed in claim 5 or 6, **characterised** in that the cellulose ether has a viscosity of 30-400 cP in a 1% water solution, as measured according to Brookfield LV, 12 rpm at 20°C.

8. Composition as claimed in any one of claims 1-7, **characterised** by having a pH of 3.0-5.5.

9. Composition as claimed in any one of claims 1-8, **characterised** in that the long-chain alkyl ester is a compound of one of the general formulae 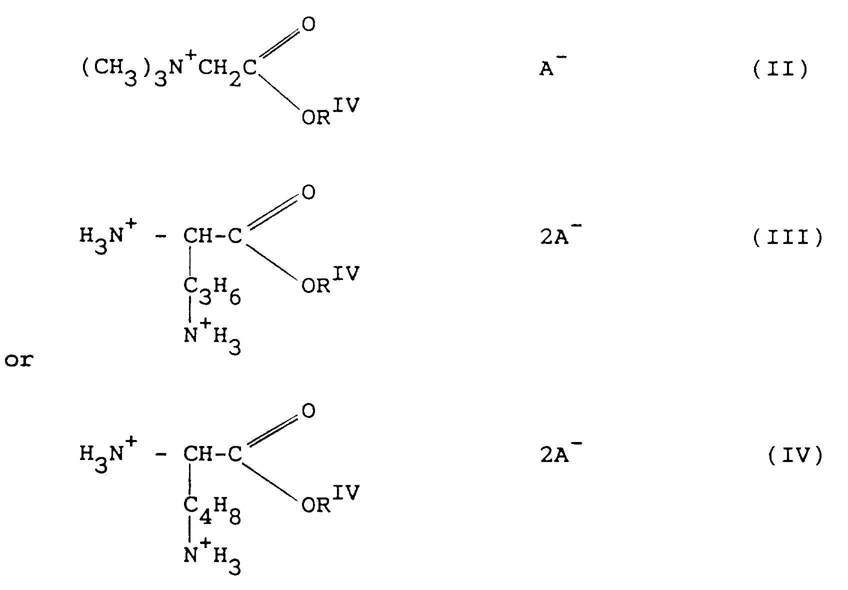 wherein R^{IV} and A are the same as above.

10. Composition as claimed in claim 9, **characterised** in that the long-chain alkyl ester is a compound of the general formula II.

11. An ethyl hydroxyethyl cellulose, **characterized** in that it has a cloud point of 30-35°C and has a viscosity of 10-10 000 cP in a 1% water solution measured according to Brookfield LV, 12 rpm at 20°C.

12. Ethyl hydroxyethyl cellulose as claimed in claim 11, **characterized** in that it has a DS_{ethyl} of 1.2-2.5 and an MS_{hydroxyethyl} of 0.5-1.5.

13. Ethyl hydroxyethyl cellulose as claimed in claim 11 or 12, **characterized** in that it has a viscosity of 30-400 cP in a 1% water solution, as measured according to Brookfield LV, 12 rpm at 20°C.

## Patentansprüche

1. Wäßrige mikrobizide Zusammensetzung mit einer mikrobiziden Wirkung in Kombination mit einer verbesserten Viskosität, dadurch gekennzeichnet, daß sie enthält
a) 1 - 30 mMol pro Liter Wasser eines langkettigen Alkylesters der allgemeinen Formel 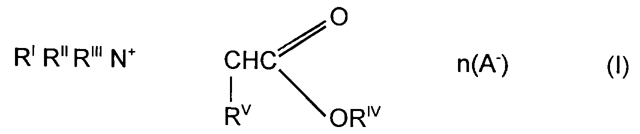 worin R^{I}, R^{II} und R^{III} Wasserstoff oder niedere Alkylgruppen sind, R^{IV} eine langkettige Alkylgruppe mit 10 - 18 Kohlenstoffatomen ist, R^{V} Wasserstoff oder eine Gruppe der Formel
R^{VI}N⁺H₃
ist, worin R^{VI} eine Alkylengruppe mit 3 - 4 Kohlenstoffatomen ist, A ein einwertiges Gegenion ist und n die Anzahl der kationischen Gruppen in der Ester-Verbindung ist, in
b) einer wäßrigen Lösung eines nicht-ionischen, wasserlöslichen Celluloseethers, die eine Viskosität von 10 - 10000 cP, gemessen gemäß Brookfield LV, 12 U/min bei 20°C, aufweist,
und daß die Zusammensetzung einen pH von unter 6 hat.

2. Zusammensetzung wie in Anspruch 1 beansprucht, dadurch gekennzeichnet, daß eine 1% Wasserlösung des Celluloseethers eine Viskosität von 10 - 10000 cP aufweist.

3. Zusammensetzung wie in irgendeinem der Ansprüche 1 und 2 beansprucht, dadurch gekennzeichnet, daß der Celluloseether einen Trübungspunkt unter 70°C hat.

4. Zusammensetzung wie in irgendeinem der Ansprüche 1 - 3 beansprucht, dadurch gekennzeichnet, daß der Celluloseether eine Alkylhydroxyalkylcellulose ist.

5. Zusammensetzung wie in Anspruch 4 beansprucht, dadurch gekennzeichnet, daß der Celluloseether eine Ethylhydroxyethylcellulose mit einem Trübungspunkt von 30 - 35°C ist.

6. Zusammensetzung wie in Anspruch 5 beansprucht, dadurch gekennzeichnet, daß der Celluloseether eine DS_{Ethyl} von 1,2 - 2,5 und eine MS_{Hydroxyethyl} von 0,5 - 1,5 hat.

7. Zusammensetzung wie in Anspruch 5 oder 6 beansprucht, dadurch gekennzeichnet, daß der Celluloseether eine Viskosität von 30 - 400 cP in einer 1% Wasserlösung, gemessen gemäß Brookfield LV, 12 U/min bei 20°C, aufweist.

8. Zusammensetzung wie in irgendeinem der Ansprüche 1 - 7 beansprucht, dadurch gekennzeichnet, daß sie einen pH von 3,0 - 5,5 hat.

9. Zusammensetzung wie in irgendeinem der Ansprüche 1 - 8 beansprucht, dadurch gekennzeichnet, daß der langkettige Alkylester eine Verbindung ist von einer der allgemeinen Formeln 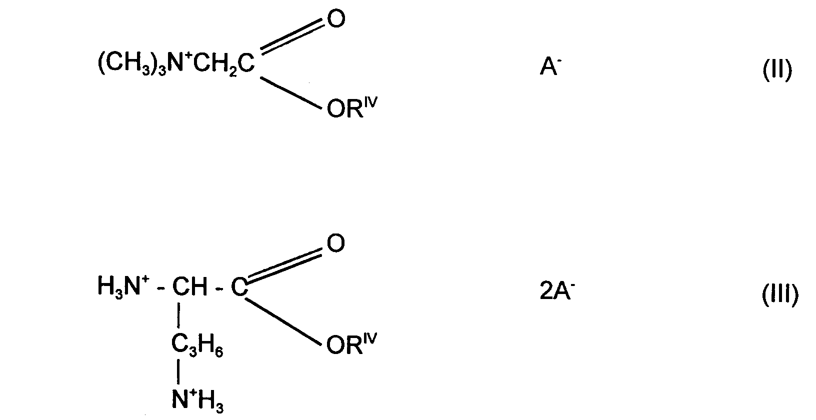 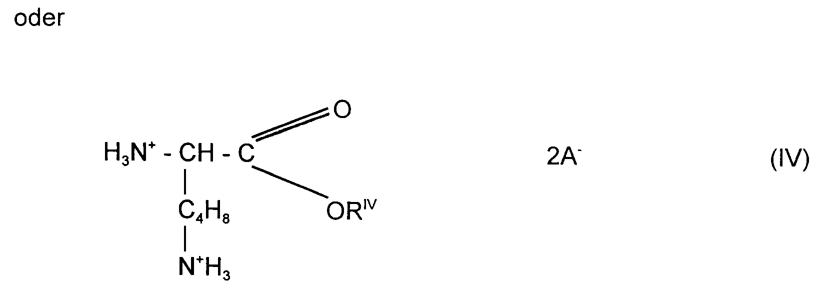 worin R^{IV} und A gleich wie oben sind.

10. Zusammensetzung wie in Anspruch 9 beansprucht, dadurch gekennzeichnet, daß der langkettige Alkylester ein Verbindung der allgemeinen Formel II ist.

11. Ethylhydroxyethylcellulose, dadurch gekennzeichnet, daß sie einen Trübungspunkt von 30 - 35°C und eine Viskosität von 10 - 10000 cP in einer 1% Wasserlösung, gemessen gemäß Brookfield LV, 12 U/min bei 20°C, aufweist.

12. Ethylhydroxyethylcellulose wie in Anspruch 11 beansprucht, dadurch gekennzeichnet, daß sie einen DS_{Ethyl} von 1,2 - 2,5 und eine MS_{Hydroxyethyl} von 0,5 - 1,5 hat.

13. Ethylhydroxyethylcellulose wie in Anspruch 11 oder 12 beansprucht, dadurch gekennzeichnet, daß sie eine Viskosität von 30 - 400 cP in einer 1% Wasserlösung, gemessen gemäß Brookfield LV, 12 U/min bei 20°C, aufweist.

## Revendications

1. Composition microbicide aqueuse ayant un effet microbicide combiné avec une viscosité améliorée, caractérisée en ce qu'elle contient
a) 1 à 30 mmoles par litre d'eau d'un ester d'alkyle à longue chaîne, de formule générale 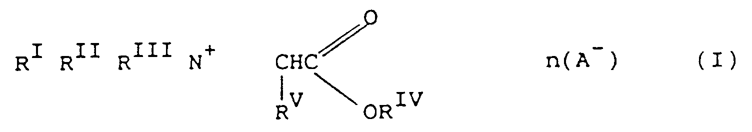 dans laquelle R^{I}, R^{II} et R^{III} sont des atomes hydrogène ou des groupes alkyle inférieurs, R^{IV} est un groupe alkyle à longue chaîne ayant 10 à 18 atomes de carbone, R^{V} est un hydrogène ou un groupe de formule
R^{VI}N⁺H₃
dans laquelle R^{VI} est un groupe alkylène ayant 3 à 4 atomes de carbone, A est un contre-ion monovalent, et n est le nombre de groupes cationiques dans le composé ester, dans
b) une solution aqueuse d'éther de cellulose hydrosoluble non ionique qui a une viscosité de 10 à 10 000 cP telle que mesurée par un appareil *Brookfield LV,* 12 tr/min. à 20°C,
et en ce que la composition a un pH inférieur à 6.

2. Composition selon la revendication 1, caractérisée en ce qu'une solution à 1 % dans l'eau de l'éther de cellulose a une viscosité de 10 à 10 000 cP.

3. Composition selon l'une quelconque des revendications 1 et 2, caractérisée en ce que l'éther de cellulose a un point de trouble inférieur à 70°C.

4. Composition selon l'une quelconque des revendications 1 à 3, caractérisée en ce que l'éther de cellulose est une alkylhydroxyalkylcellulose.

5. Composition selon la revendication 4, caractérisée en ce que l'éther de cellulose est une éthylhydroxyéthylcellulose ayant un point de trouble de 30 à 35°C.

6. Composition selon la revendication 5, caractérisée en ce que l'éther de cellulose a un DS_{éthyle} de 1,2 à 2,5 et un MS_{hydroxyéthyle} de 0,5 à 1,5.

7. Composition selon la revendication 5 ou 6, caractérisée en ce que l'éther de cellulose a une viscosité de 30 à 400 cP en une solution à 1 % dans l'eau, telle que mesurée par un appareil *Brookfield LV,* 12 tr/min. à 20 °C.

8. Composition selon l'une quelconque des revendications 1 à 7, caractérisée en ce qu'elle a un pH de 3,0 à 5,5.

9. Composition selon l'une quelconque des revendications 1 à 8, caractérisée en ce que l'ester d'alkyle à longue chaîne est un composé de l'une des formules générales suivantes 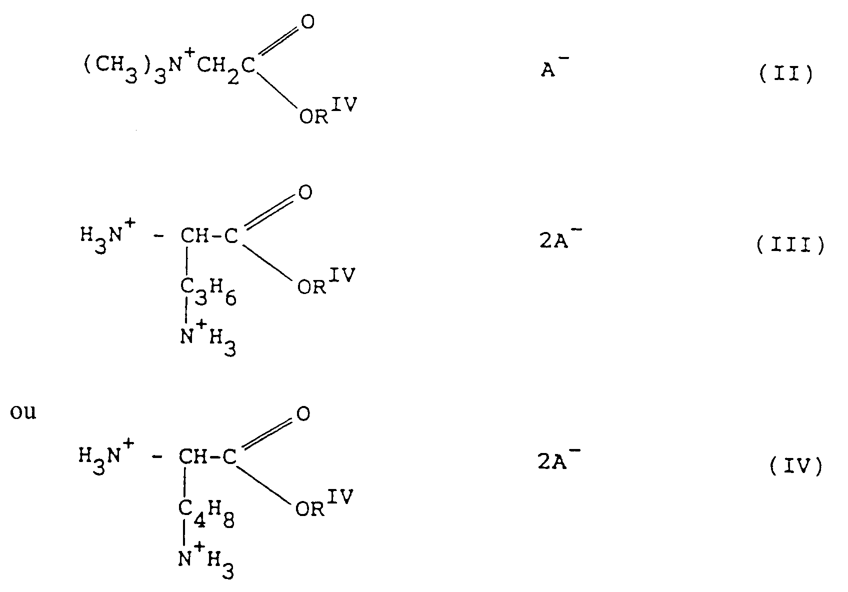 dans lesquelles R^{IV} et A sont les mêmes que ci-dessus.

10. Composition selon la revendication 9, caractérisée en ce que l'ester d'alkyle à longue chaîne est un composé de formule générale II.

11. Ethylhydroxyéthylcellulose, caractérisée en ce qu'elle a un point de trouble de 30 à 35 °C et une viscosité de 10 à 10 000 cP en une solution à 1 % dans l'eau, mesurée par un appareil *Brookfield LV,* 12 tr/min. à 20 °C.

12. Ethylhydroxyéthylcellulose selon la revendication 11, caractérisée en ce qu'elle a un DS_{éthyle} de 1,2 à 2,5 et un MS_{hydroxyéthyle} de 0,5 à 1,5.

13. Ethylhydroxyéthylcellulose selon la revendication 11 ou 12, caractérisée en ce qu'elle a une viscosité de 30 à 400 cP en une solution à 1 % dans l'eau, telle que mesurée par un appareil *Brookfield LV,* 12 tr/min. à 20 °C.
